# EUROPÄISCHE PATENTSCHRIFT

(11) **EP 1 596 730 B1**
(45) Veröffentlichungstag und Bekanntmachung des Hinweises auf die Patenterteilung: **21.09.2011**
(21) Anmeldenummer: 04710838.6
(22) Anmeldetag: 13.02.2004
(51) Int. Cl.: A61B 17/15, A61B 17/17

(54) **CHIRURGISCHE POSITIONIER- UND HALTEVORRICHTUNG**
SURGICAL POSITIONING AND RETAINING DEVICE
DISPOSITIF CHIRURGICAL DE POSITIONNEMENT ET DE MAINTIEN

(30) Priorität: 28.02.2003 DE 10309987
(43) Veröffentlichungstag der Anmeldung: 23.11.2005
(73) Patentinhaber: Aesculap AG, 78532 Tuttlingen (DE)
(72) Erfinder: HAGEN, Thomas, 78532 Tuttlingen (DE); REICH, Jan, 78073 Hochemmingen (DE)
(74) Vertreter: Pioch, Holger
(86) Internationale Anmeldenummer: PCT/EP2004/001347
(87) Internationale Veröffentlichungsnummer: WO 2004/075763

(56) Entgegenhaltungen:
- DE-A- 19 802 751
- FR-A- 2 806 901
- US-A- 4 457 307
- US-A- 5 395 376

## Beschreibung

Die vorliegende Erfindung betrifft eine chirurgische Positionier- und Haltevorrichtung zum Positionieren und Halten einer Führung für ein chirurgisches Bearbeitungswerkzeug, mit mindestens einem Befestigungselement zum Fixieren in einem zu bearbeitenden Knochen, mit mindestens einem Bearbeitungswerkzeug und mit einer an dem mindestens einen Befestigungselement gehaltenen Plattform zum Halten der Führung, wobei die Führung um eine erste Drehachse drehbar gelagert und derart ausgebildet ist, dass mit dem in der Führung geführten oder an dieser gehaltenen Bearbeitungswerkzeug mindestens eine zur ersten Drehachse konzentrische Fläche präparierbar ist.

Derartige Vorrichtungen werden beispielsweise bei Gelenkoperationen verwendet, bei denen Teile eines beschädigten Gelenks durch künstliche Gelenkteile ersetzt werden. Zu diesem Zweck wird die Vorrichtung mit dem mindestens einen Befestigungselement an dem zu bearbeitenden Knochen fixiert und eine Verankerungsfläche für das künstliche Gelenkteil mittels eines in der Führung geführten Bearbeitungswerkzeugs präpariert. Dabei werden insbesondere ebene Verankerungsflächen an Knochen durch teilweise Resektion geformt. Ein Beispiel zum Präparieren einer sphärischen Verankerungsfläche ist in der US 5,314,482 beschrieben. Nachteilig dabei ist, dass das Bearbeitungswerkzeug von vorn an den zu bearbeitenden Knochen herangeführt werden muss, was ein vollständiges Eröffnen des beschädigten Gelenks erforderlich macht.

Ein Verfahren und eine Vorrichtung zum Präparieren eines Femurs vor dem Implantieren einer Kniegelenkendoprothese sind aus der US 5,395,376 bekannt.

Daher ist es Aufgabe der vorliegenden Erfindung, eine chirurgische Positionier- und Haltevorrichtung der eingangs beschriebenen Art so zu verbessern, dass Verankerungsflächen an einem zu bearbeitenden Knochen auf einfache Weise mit hoher Präzision präpariert werden können.

Diese Aufgabe wird bei einer chirurgischen Positionier- und Haltevorrichtung der eingangs beschriebenen Art erfindungsgemäß dadurch gelöst, dass das Bearbeitungswerkzeug ein Stirnfräser ist und dass an der Vorrichtung ein Referenzelement zur Navigationskontrolle vorgesehen ist.

Mit einer solchen Vorrichtung lassen sich auf einfachste Weise zylindrische Oberflächen an einem Knochen präparieren. Durch die besondere Orientierung der Drehachse ist es möglich, beispielsweise an einem Femur eine Kondyle von lateral oder medial kommend zu bearbeiten. Eine komplette Eröffnung des zu bearbeitenden Gelenks ist daher nicht erforderlich, vielmehr läßt sich ein solcher chirurgischer Eingriff auch minimalinvasiv oder durch eine Miniarthrotomie ausführen. Von Vorteil ist es, dass an der Vorrichtung ein Referenzelement zur Navigationskontrolle vorgesehen ist. Damit lassen sich navigationsgestützt Flächen an dem zu bearbeitenden Knochen präparieren. Insbesondere dann, wenn bereits das mindestens eine Befestigungselement navigationsgestützt an dem zu bearbeitenden Knochen verankert wird, kann eine Feinjustierung der ersten Drehachse relativ zu dem mindestens einen Befestigungselement unter Navigationskontrolle vorgenommen werden.

Um einen besonders sicheren Halt der Vorrichtung am zu bearbeitenden Knochen zu gewährleisten, ist es günstig, wenn ein zweites Befestigungselement zum Fixieren in dem zu bearbeitenden Knochen vorgesehen ist und wenn das zweite Befestigungselement an der Plattform geführt und/oder gehalten ist.

Um die Plattform auf einfache Weise mit dem mindestens einen Befestigungselement zu verbinden, kann die Plattform mindestens eine Befestigungselementaufnahme zum Aufnehmen des mindestens einen Befestigungselements aufweisen. Das Befestigungselement kann in die Befestigungselementaufnahme eingeführt und in dieser wahlweise zusätzlich gegen eine Relativbewegung gesichert werden.

Eine besonders einfache Ausgestaltung der Vorrichtung ergibt sich, wenn die mindestens eine Befestigungselementaufnahme eine Bohrung umfasst. Insbesondere kann es sich dabei um eine Sacklochbohrung handeln.

Um die Plattform auf einfache Weise mit mehreren Befestigungselementen zu verbinden, ist es vorteilhaft, wenn Längsachsen von mindestens zwei Befestigungselementaufnahmen parallel zueinander ausgerichtet sind. Die Plattform kann dann auf in dem zu bearbeitenden Knochen festgelegte Befestigungselemente in Richtung der Längsachsen in oder auf die Befestigungselementaufnahmen geführt werden.

Gemäß einer bevorzugten Ausführungsform der Erfindung kann vorgesehen sein, dass die Längsachsen der mindestens zwei Befestigungselementaufnahmen parallel oder nahezu parallel zu der ersten Drehachse verlaufen. Wird beispielsweise das mindestens eine Befestigungselement navigationsgestützt im zu bearbeitenden Knochen verankert, läßt sich so eine Richtung der ersten Drehachse genau oder grob vorgeben.

Vorteilhaft ist es, wenn an der Plattform eine Lagerwelle vorgesehen ist und wenn die Lagerwelle die erste Drehachse definiert. Die erste Drehachse ist auf diese Weise optisch sofort erkennbar. Die Lagerwelle kann relativ zur Plattform beweglich oder an dieser feststehend angeordnet sein.

Um eine Feinjustage der ersten Drehachse relativ zu dem mindestens einen Befestigungselement zu gestatten, ist die Lagerwelle relativ zu dem mindestens einen Befestigungselement in einer ersten Verschieberichtung an der Plattform verschiebbar gelagert. Die erste Verschieberichtung kann beliebig gewählt werden, insbesondere parallel oder quer zur ersten Drehachse.

Zur Feineinstellung der ersten Drehachse relativ zu dem mindestens einen Befestigungselement, kann die Lagerwelle relativ zu dem mindestens einen Befestigungselement in einer zweiten Verschieberichtung an der Plattform verschiebbar gelagert sein. Insbesondere können die erste und die zweite Verschieberichtung rechtwinklig zueinander orientiert sein.

Eine Relativposition der ersten Drehachse zu dem mindestens einen Befestigungselement ist für einen dritten Freiheitsgrad justierbar, wenn die Lagerwelle relativ zu dem mindestens einen Befestigungselement um eine zweite Drehachse an der Plattform schwenkbar gelagert ist. Damit läßt sich ein Neigungswinkel der ersten Drehachse relativ zu einer Längsachse des mindestens einen Befestigungselements verstellen.

Vorzugsweise sind die erste und die zweite Drehachse rechtwinklig zueinander orientiert. Dadurch läßt sich die erste Drehachse relativ zu dem mindestens einen Befestigungselement neigen, so dass die zu präparierende Fläche am zu bearbeitenden Knochen relativ zu einer Längsachse des mindestens einen Befestigungselements geneigt sein kann.

Ein besonders einfacher Aufbau der Vorrichtung ergibt sich, wenn diese einen um die erste Drehachse drehbar gelagerten Gelenkarm umfasst, wenn ein Ende des Gelenkarms an der Lagerwelle um die erste Drehachse drehbar gelagert ist und wenn ein anderes Ende des Gelenkarms die Führung trägt. Mit einem derartigen Gelenkarm läßt sich auf besonders einfache Weise eine zirkelartige Konstruktion der Vorrichtung verwirklichen.

Einen besonders guten Halt und eine besonders gute Führung des Bearbeitungswerkzeugs an der Vorrichtung ergibt sich, wenn die Führung eine Hülse zum Aufnehmen des Bearbeitungswerkzeugs umfasst. Beispielsweise kann ein Bearbeitungswerkzeug in Form eines Fräsers oder eines Bohrers nahezu spielfrei in der Hülse geführt werden, so dass zylindrische Flächen am Knochen hochpräzise präpariert werden können.

Günstig ist es, wenn die Hülse am Gelenkarm rotierbar gelagert ist. Dadurch läßt sich ein Verschleiß der Vorrichtung verringern. Vorteilhafterweise ist die Hülse am Gelenkarm kugelgelagert.

Auf einfache Weise läßt sich die erste Drehachse relativ zu dem mindestens einen Befestigungselement in ihrer Position variieren, wenn an der Plattform ein erster Linearantrieb vorgesehen ist zum Verschieben der Lagerwelle relativ zu dem mindestens einen Befestigungselement in der ersten Verschieberichtung.

Ein besonders einfacher Aufbau der Vorrichtung ergibt sich, wenn der erste Linearantrieb ein Spindeltrieb mit einer ersten Gewindespindel und einem ersten Antriebsknopf ist und wenn eine Längsachse der ersten Gewindespindel die erste Verschieberichtung definiert. Für einen Spindeltrieb wird nur eine minimale Zahl an Bauteilen benötigt, wodurch die Konstruktion der Vorrichtung vereinfacht wird.

Vorteilhaft ist es, wenn an der Plattform ein zweiter Linearantrieb vorgesehen ist zum Verschieben der Lagerwelle relativ zu dem mindestens einen Befestigungselement in der zweiten Verschieberichtung. Mit dem zweiten Linearantrieb läßt sich eine Position der ersten Drehachse relativ zum mindestens einen Befestigungselement auf einfache Weise verstellen.

Zur Vereinfachung einer Konstruktion der Vorrichtung ist es günstig, wenn der zweite Linearantrieb ein zweiter Spindeltrieb mit einer zweiten Gewindespindel und einem zweiten Antriebsknopf ist und wenn eine Längsachse der zweiten Gewindespindel die zweite Verschieberichtung definiert.

Um den Aufbau der Vorrichtung besonders kompakt zu gestalten, kann die Längsachse der zweiten Gewindespindel die zweite Drehachse definieren. Beispielsweise könnte die Gewindespindel als Lagerwelle für eine Schwenkbewegung um die zweite Drehachse dienen.

Um eine Schwenkbewegung der ersten Drehachse relativ zu dem mindestens einen Befestigungselement auf einfache Weise zu verwirklichen, kann an der Plattform ein Exzenterantrieb vorgesehen sein zum Verschwenken der Lagerwelle relativ zu dem mindestens einen Befestigungselement um die zweite Drehachse.

Eine besonders einfache Konstruktion ergibt sich, wenn der Exzenterantrieb einen exzentrisch um eine dritte Drehachse gelagerten Drehkörper umfasst und wenn die dritte Drehachse parallel zur zweiten Drehachse verläuft.

Beispielsweise bei Kniegelenksoperationen kann sich das Problem ergeben, dass die erste Drehachse so positioniert werden muss, dass sie einen Ansatzbereich von Seitenbändern, Muskeln, Sehnen oder diese selbst schneidet. Würde daher durch das mindestens eine Befestigungselement die erste Drehachse vorgegeben, hätte dies eine Beschädigung der Seitenbänder zur Folge. Daher ist es günstig, wenn das mindestens eine Befestigungselement von der Drehachse beabstandet ist. Insbesondere kann die Vorrichtung so ausgestaltet sein, dass die Befestigungselemente in einem vom Ansatzbereich der Seitenbänder entfernten Bereich des zu bearbeitenden Knochens angeordnet werden können, so dass keine Sehnen, Muskeln oder Bänder beschädigt werden. Trotzdem kann bei dieser Konstruktion die erste Drehachse Bänder oder dergleichen schneiden.

Gemäß einer bevorzugten Ausführungsform der Erfindung kann es vorteilhaft sein, wenn die Führung relativ zur Plattform in einer Drehstellung feststellbar ist. Die Führung kann dann je nach Ausgestaltung selbst eine Drehachse für ein Bearbeitungswerkzeug definieren, beispielsweise ein zylindrisch gekrümmtes Sägeblatt, mit welchem ebenfalls eine zylindrische Oberfläche an einem zu bearbeitenden Knochen präpariert werden kann.

Insbesondere ist es vorteilhaft, wenn die Führung eine vierte Drehachse definiert. Auf diese Weise lassen sich mit entsprechenden Bearbeitungswerkzeugen, beispielsweise mit zylindrisch gekrümmten Sägeblättern, zur vierten Drehachse konzentrische Flächen an einem zu bearbeitenden Kochen präparieren.

Eine besonders kompakte Bauform ergibt sich, wenn eine Breite der Plattform in der zweiten Verschieberichtung maximal 30 mm beträgt. Damit eignet sich die Vorrichtung auch für minimal invasive Eingriffe.

Vorteilhaft ist es, wenn ein Abstand der Führung von der ersten Drehachse in einem Bereich von 15 mm bis 50 mm liegt. Damit lassen sich Krümmungsradien der zu bearbeitenden Oberfläche in dem angegebenen Bereich verwirklichen oder bei entsprechendem Durchmesser des Bearbeitungswerkzeugs sogar noch kleinere. Außerdem wird so eine Baugröße der Vorrichtung verringert.

Um die Vorrichtung besonders universell einsetzen zu können, kann gemäß einer bevorzugten Ausführungsform der Erfindung ein Satz unterschiedlich langer Gelenkarme vorgesehen sein und kann jeder Gelenkarm einen anderen Abstand zwischen der Drehachse und der Führung aufweisen. Je nach Größe des zu bearbeitenden Knochen kann ein optimal langer Gelenkarm ausgewählt und mit der Plattform zum Führen des Bearbeitungswerkzeugs verbunden werden.

Günstig ist es, wenn die Führung in einer Richtung parallel zur ersten Drehachse relativ zur Plattform verschiebbar ist. Eine derartige Anordnung ermöglicht eine weitere Einstellmöglichkeit der Führung relativ zu den Befestigungselementen. Insbesondere dann, wenn eine Verstellbarkeit der Lagerwelle relativ zu den Befestigungspins nicht oder nur erschwert möglich ist, läßt sich auf diese Weise die Führung in eine gewünschte Position bringen.

Vorteilhaft ist es, wenn die von der Führung definierte vierte Drehachse rechtwinklig zur ersten Drehachse verläuft. Diese Anordnung ermöglicht es, einen Knochen mit einem Bearbeitungswerkzeug von vorn zu bearbeiten, beispielsweise mit einem Stirnfräser. Auf diese Weise läßt sich eine zur ersten Drehachse konzentrische Fläche herstellen.

Vorzugsweise schneiden sich die vierte und die erste Drehachse. Dadurch kann mit einem Ende eines Bearbeitungswerkzeugs direkt eine zur ersten Drehachse konzentrischen Bahn beschrieben werden.

Die nachfolgende Beschreibung bevorzugter Ausführungsformen der vorliegenden Erfindung dient im Zusammenhang mit der Zeichnung der näheren Erläuterung. Es zeigen:
- Figur 1:: eine perspektivische Ansicht eines an einem zu bearbeitenden Kno- chen festgelegten nicht erfindungsgemäßen Ausrichtinstruments;
- Figur 2:: eine laterale Ansicht des an dem zu bearbeitenden Knochen festge- legten Ausrichtinstruments;
- Figur 3:: eine Schnittansicht des Ausrichtinstruments;
- Figur 4:: eine Schnittansicht des Ausrichtinstruments längs Linie 4-4 in Figur 3;
- Figur 5:: eine Schnittansicht des Ausrichtinstruments längs Linie 5-5 in Figur 3;
- Figur 6:: eine Schnittansicht ähnlich Figur 3 mit dem Ausrichtinstrument in einer verschwenkten Stellung;
- Figur 7:: eine perspektivische Ansicht eines zweiten Ausführungsbeispiels eines Ausrichtinstruments;
- Figur 8:: eine weitere perspektivische Ansicht des zweiten Ausführungsbei- spiels eines Ausrichtinstruments; und
- Figur 9:: eine Querschnittansicht des zweiten Ausführungsbeispiels eines Aus- richtinstruments.

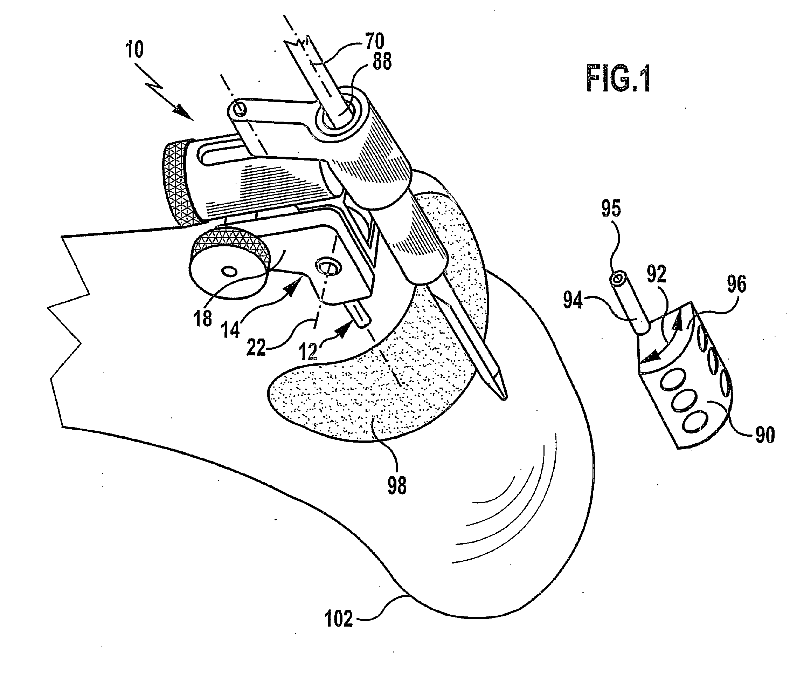

In Figur 1 ist eine nicht erfindungsgemäße chirurgische Positionier- und Haltevorrichtung dargestellt, welche ein insgesamt mit dem Bezugszeichen 10 versehenes Ausrichtinstrument und zwei Knochenpins 12 umfasst.

Das Ausrichtinstrument 10 umfasst zwei relativ zueinander schwenkbar gelagerte Rahmenteile, nämlich einen mit den Knochenpins 12 verbundenen Halterahmen 14 und einen Lagerrahmen 16. Der Halterahmen 14 weist zwei parallel zueinander angeordnete, flache, L-förmige, über eine Verbindungsplatte 20 miteinander verbundene Seitenwände 18 auf, zwischen denen der insgesamt im wesentlichen L-förmige Lagerrahmen 16 um eine Schwenkachse 22 relativ zum Halterahmen 14 gelagert ist.

Die Schwenkachse 22 wird definiert durch eine drehfest mit den Seitenwänden 18 verbundene Gewindespindel 24 eines insgesamt mit dem Bezugszeichen 25 definierten Spindeltriebs, die in einem Bereich zwischen den beiden Seitenwänden 18 mit einem Außengewinde versehen ist. Sie durchsetzt ferner eine Bohrung 26 eines zwischen den Seitenwänden 18 gehaltenen Schenkels 28 des Lagerrahmens 16. Der Schenkel 28 ist quer zur Bohrung 26 mit einer quaderförmigen Durchbrechung 30 versehen, in welcher ein mit einem Innengewinde 34 versehenes Stellrad 32 angeordnet ist. Das Innengewinde 34 korrespondiert zu einem Außengewinde 36 der Gewindespindel 24. Eine Breite des Schenkels 28 in Richtung der Schwenkachse 22 ist kleiner als ein Abstand zwischen den Seitenwänden 18, so dass mittels des Spindeltriebs 25, also durch Verdrehen des Stellrads 32 auf der Gewindespindel 24, eine seitliche Bewegung des Lagerrahmens 16 relativ zum Halterahmen 14 ermöglicht wird. Die Gewindespindel 24 in Verbindung mit dem Stellrad 32 bildet somit einen Linearantrieb in Form des Spindeltriebs 25.

Eine hohlzylindrische Lagerhülse 38 bildet einen zweiten, zum Schenkel 28 rechtwinklig verlaufenden Schenkel des Lagerrahmens 16. Eine Symmetrieachse 40 der Lagerhülse 38 ist senkrecht zur Schwenkachse 22 orientiert. Parallel zur Symmetrieachse 40 ist die Lagerhülse 38 mit einem sich nahezu über die gesamte Länge der Lagerhülse 38 erstreckenden Längsschlitz 42 versehen, durch den ein zylindrischer Lagerbolzen 44 hervorsteht. Er ist drehfest mit einem zylindrischen Verschiebekörper 46 verbunden, welcher in der Lagerhülse 38 geführt ist. Ein Außendurchmesser des Verschiebekörpers 46 ist nur unwesentlich kleiner als ein Innendurchmesser der Lagerhülse 38, so dass der Verschiebekörper 46 nur in Richtung der Symmetrieachse 40 in der Lagerhülse 38 verschoben werden kann. Eine Rotation des Verschiebekörpers 46 in der Lagerhülse 38 wird durch den den Längsschlitz 42 durchsetzenden Lagerbolzen 44 verhindert.

Der Verschiebekörper 46 ist ferner drehfest mit einem Gewindebolzen 48 versehen, der durch eine Stirnbohrung 50 einer Stirnfläche 52 der Lagerhülse 38 hervorsteht. In der Stirnbohrung 50 ist formschlüssig eine mit einem Innengewinde versehene Gewindehülse 54 eingesetzt, welche drehfest mit einem Rändelkopf 56 verbunden ist, welcher außen an der Stirnfläche 52 anliegt. Die Gewindehülse 54 ragt etwas in die Gewindehülse 38 hinein und ist mit einem Sprengring 58 gegen eine axiale Verschiebung in Richtung der Symmetrieachse 40 gesichert. Die Symmetrieachse 40 fällt mit einer Symmetrieachse des Gewindebolzens 48 zusammen. Durch eine Drehung des Rändelkopfs 56 wird der Gewindebolzen 48 in Richtung der Symmetrieachse 40 bewegt, so dass der Verschiebekörper 46 in der Lagerhülse 38 linear verschoben wird. Auf diese Weise wird ein Linearantrieb 60 in Form eines Spindeltriebs gebildet.

Ein quaderförmiger Lenker 62 ist an seinem einen Ende mit einer Bohrung 64 versehen, in welche der Lagerbolzen 44 eingeführt ist. Dadurch läßt sich der Lenker 62 um den eine Lagerwelle bildenden Lagerbolzen 44 verschwenken, und zwar um eine durch den Lagerbolzen 44 definierte Drehachse 66. An seinem anderen Ende ist der Lenker 62 einstückig mit einer Führungshülse 68 verbunden, deren Symmetrieachse eine Rotationsachse 70 definiert. Die Rotationsachse 70 verläuft parallel zur Drehachse 66. In die Führungshülse 68 ist eine weitere Lagerhülse 72 eingesetzt, deren Symmetrieachse mit der Rotationsachse 72 zusammenfällt. Die Lagerhülse 72 ist etwas mehr als doppelt so lang als die Führungshülse 68. Sie ist drehfest mit der Führungshülse 68 verbunden. Denkbar wäre auch eine Lagerung der Lagerhülse mittels eines Kugellagers an der Führungshülse 68.

Parallel zur Gewindespindel 24 ist ein Exzenterbolzen 74 in Bohrungen 76 in den Seitenwänden 18 drehbar gehalten. Zwischen den Seitenwänden 18 ist ein drehfest mit dem Exzenterbolzen 74 verbundener Stützzylinder 78 angeordnet. Ein Ende des Exzenterbolzens 74 ist mit einem Stellrad 80 angeordnet. Ein ein anderes Ende mit einem Bolzenkopf 82 versehen. Eine Bewegung in Richtung einer von einer Längsachse des Exzenterbolzens 74 definierten Exzenterachse 84 wird verhindert, denn der Exzenterbolzen 74 wird beidseitig an einer Seitenwand 18 durch den Bolzenkopf 82 und den Stützzylinder 78, an der anderen Seitenwand 18 durch den Stützzylinder 78 und das Stellrad 80 gehalten. Eine Umfangswandung des Stützzylinders 78 bildet eine Stützfläche 86 für die Lagerhülse 38. Durch Verdrehen des Stellrads 80 wird der Stützzylinder 78 um die Exzenterachse 84 verschwenkt, so dass sich ein Abstand der am Stützzylinder 78 anliegenden Lagerhülse 38 von der Exzenterachse 84 verändert, wodurch eine Schwenkbewegung der Lagerhülse 38 und damit des Lagerrahmens 16 um die Schwenkachse 22 bewirkt wird. Durch dieses Verschwenken wird auch die Drehachse 66 relativ zur Schwenkachse 22 geneigt.

In der Lagerhülse 72 kann ein Bearbeitungswerkzeug geführt werden, beispielsweise ein Fräser 88 oder ein Sägeblatt 90. Das Sägeblatt 90 ist in Form eines Zylinderwandabschnitts gekrümmt und erstreckt sich über einen Winkelbereich 92 von etwa 100°. Konzentrisch zum Sägeblatt 90 ist ein Haltezapfen 94 an einer Deckplatte 96 angeordnet. Der Haltezapfen 94 kann ebenfalls in der Lagerhülse 72 geführt werden.

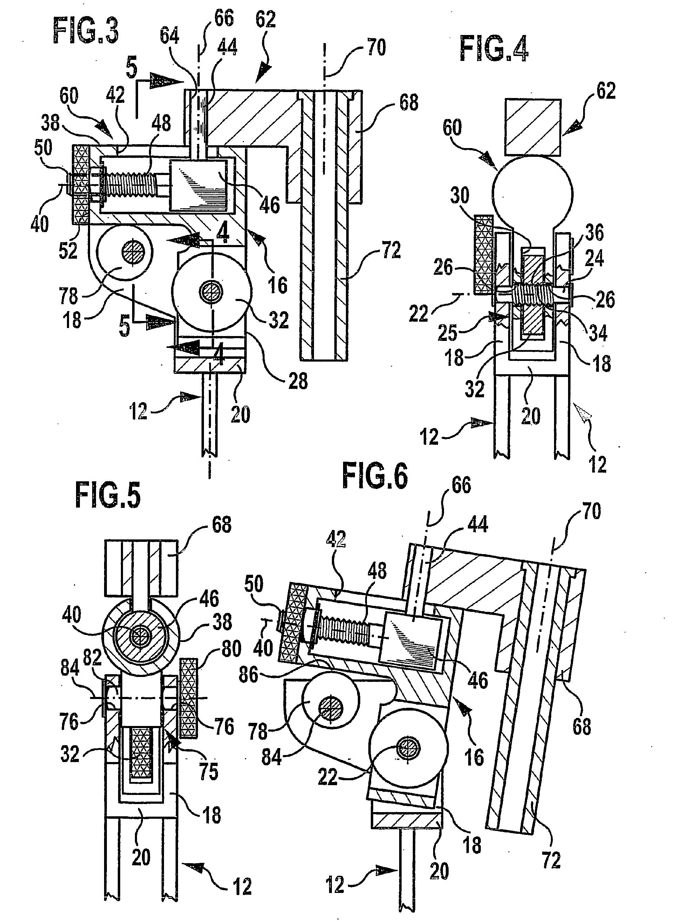

Nachfolgend wird in Verbindung mit den Figuren 1 bis 6 die Anwendung des Ausrichtinstruments 10 beispielhaft in Verbindung mit einer Präparierung einer zylindrischen Verankerungsfläche 98 zum Verankern einer künstlichen Kondyle 100 erläutert, welche eine teilweise geschädigte natürliche Kondyle an einem Femur 102 ersetzt.

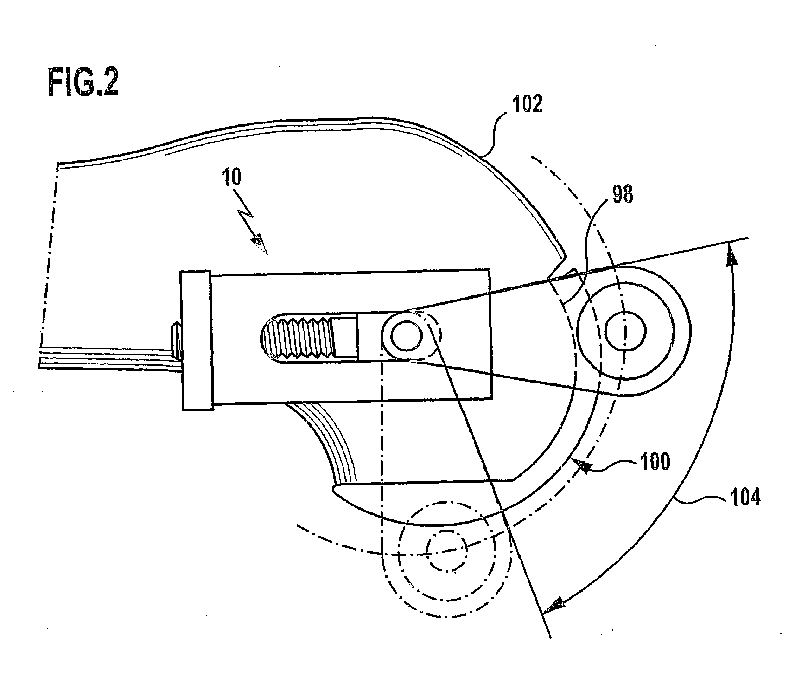

Das Ausrichtinstrument 10 wird mit den beiden Knochenpins 12 entweder lateral oder medial, je nach zu ersetzender geschädigter Kondyle, an den Femur 102 herangeführt. Dies kann navigationsgestützt erfolgen. Die Knochenpins 12 werden in den Femur 102 eingeschlagen und das Ausrichtinstrument auf diese Weise verankert. Ein Bearbeitungswerkzeug, beispielsweise der Fräser 88, wird in die Lagerhülse 72 eingeführt und zur Bearbeitung des Femurs 102 in Rotation versetzt und gleichzeitig durch Verschwenken des Lenkers 62 um die Drehachse 66, wie in Figur 2 dargestellt, in einem Winkelbereich 104 von etwa 80 bis 90° verschwenkt. Auf diese Weise wird der Femur 102 in gewünschter Weise reseziert.

Falls erforderlich, kann vor dem Präparieren der Verankerungsfläche 98 eine Position der Drehachse 66 relativ zum Femur 102 nachjustiert werden. Hierzu kann mittels des Linearantriebs 60 eine Verstellung in Richtung der Symmetrieachse 40 vorgenommen werden. Ferner kann eine lineare Verschiebung des Lagerbolzens 44 relativ zu den Knochenpins 12 durch Drehen am Stellrad 32 in Richtung der Schwenkachse 22 erfolgen.

Außerdem läßt sich die Drehachse 66 in ihrer Neigung relativ zu den Knochenpins 12 verändern durch Drehen am Stellrad 80, was, wie oben beschrieben, eine Verschwenkung des Lagerrahmens 16 um die Schwenkachse 22 bewirkt.

Zur Verwendung mit dem oben beschriebenen zylindrischen Sägeblatt 90 kann der Lenker 62 in einer Schwenkstellung festgelegt werden. Bei alternativ geformten Sägeblättern 90 kann auch eine den Haltezapfen 94 in dessen Längsrichtung durchsetzende Zapfenbohrung 95 vorgesehen sein, die den Lagerbolzen 44 aufnehmen kann. Anstelle des Lenkers 62 läßt sich so der Haltezapfen 94 am Lagerbolzen 44 schwenkbar lagern.

Um Verankerungsflächen mit unterschiedlichen Radien zu erhalten, sind mehrere verschiedene Lenker 62 vorgesehen, bei denen jeweils der Abstand zwischen der Drehachse 66 und der Rotationsachse 70 variiert. Je nach zu implantierender Kondyle 100 wird ein entsprechender Lenker 62 zur Präparation der Verankerungsfläche 98 ausgewählt.

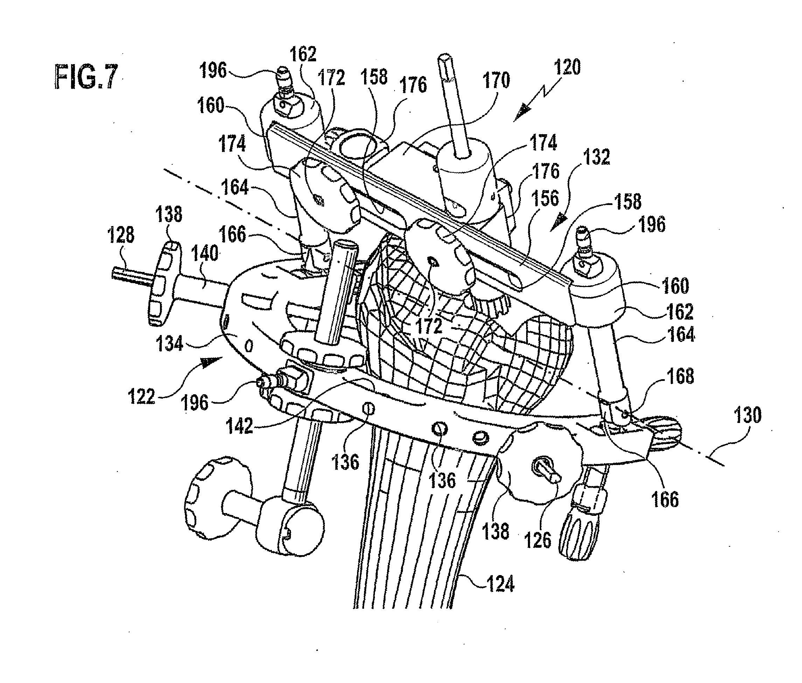

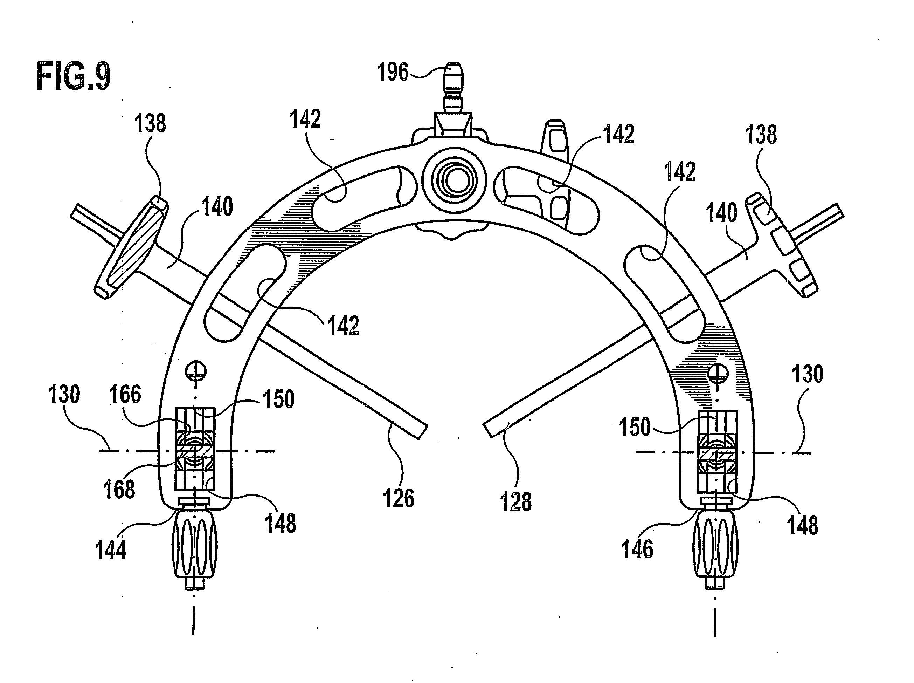

Ein zweites, insgesamt mit dem Bezugszeichen 120 versehenes Ausrichtinstrument ist in den Figuren 7 bis 9 dargestellt. Es umfasst eine insgesamt mit dem Bezugszeichen 122 versehene Plattform, die an einem Femur 124 mittels zweier Knochenpins 126 und 128, die zwischen sich in etwa einen Winkel von 120° einschließen, gehalten werden kann. An der Plattform um eine Schwenkachse 130 schwenkbar gelagert ist ein insgesamt mit dem Bezugszeichen 132 versehener Schwenkbügel.

Sowohl die Plattform 122 als auch der Schwenkbügel 132 sind im wesentlichen symmetrisch bezüglich einer zur Schwenkachse 130 senkrechten Symmetrieebene.

Die Plattform 122 umfasst einen halbringförmigen Rahmen 134, der eine Rahmenebene definiert. Parallel zur Rahmenebene sind eine Mehrzahl von Pinlöchern 136 vorgesehen, durch welche die Knochenpins 126 und 128 durchsteckbar sind, so dass diese unter einem Winkel von 120° relativ zueinander in einer Ebene ausgerichtet sind. Vom Femur 124 abstehende Enden der Knochenpins 126 und 128 sind mit einem Schraubengewinde versehen, auf welches eine mit einem Drehknopf 138 versehene Gewindehülse 140 aufschraubbar ist und den Rahmen 134 an den beiden Knochenpins 126 und 128 fixiert. Quer zu Längsachsen der Knochenpins 126 und 128 sind am Rahmen 134 langlochartige Öffnungen 142 vorgesehen. Freie Enden 144 und 146 des Rahmens 134 weisen eine sich von den Enden 144 und 146 weg erstreckende längliche Öffnung 148 auf. In diese ist jeweils ein sich von den Enden 144 und 146 weg erstreckender Schaft 150 eingesetzt, der eine auf dem Schaft verschiebbare Lagerhülse 152 trägt und eine Längsachse 151 definiert.

An der Lagerhülse 152 ist rechtwinklig abstehend ein Lagerbolzen 154 angeordnet, an welchem der im wesentliche U-förmige Schwenkbügel 132 um die Schwenkachse 130 schwenkbar gelagert ist. Der Schwenkbügel 132 umfasst eine langgestreckte quaderförmige Platte 156, welche mit zwei symmetrisch angeordneten Langlöchern 158 versehen ist.

An freien Enden 160 der Platte 156 ist ein Zylinder 162 mit seiner Längsachse quer zur Längsrichtung der Platte 156 angeordnet. Er setzt sich in einem im Durchmesser verringerten, zylindrischen Stab 164 fort, welcher an seinem freien Ende eine Lagernut 166 trägt, welche mittels eines Gelenkstifts 168 am Lagerbolzen 154 um die Schwenkachse 130 schwenkbar gelagert ist.

Parallel zu den Langlöchern 158 und zur Längsrichtung der Platte 156 verschiebbar ist ein quaderförmiger Lagerschlitten 170. Er trägt zwei die Langlöcher 158 parallel durchsetzende Gewindestifte 172, auf welche jeweils eine Rändelmutter 174 aufgeschraubt ist. Mittels der Rändelmuttern 174 läßt sich der Lagerschlitten 170 an der Platte 156 festklemmen. Um eine Position des Lagerschlittens 170 relativ zur Platte 156 zu verändern, werden die beiden Rändelmuttern 174 gelöst und der Lagerschlitten 170 relativ zur Platte 156 verschoben, bis eine gewünschte Stellung erreicht ist. Mittels der Rändelmuttern 174 kann der Lagerschlitten 170 dann wieder an der Platte 156 klemmend festgelegt werden.

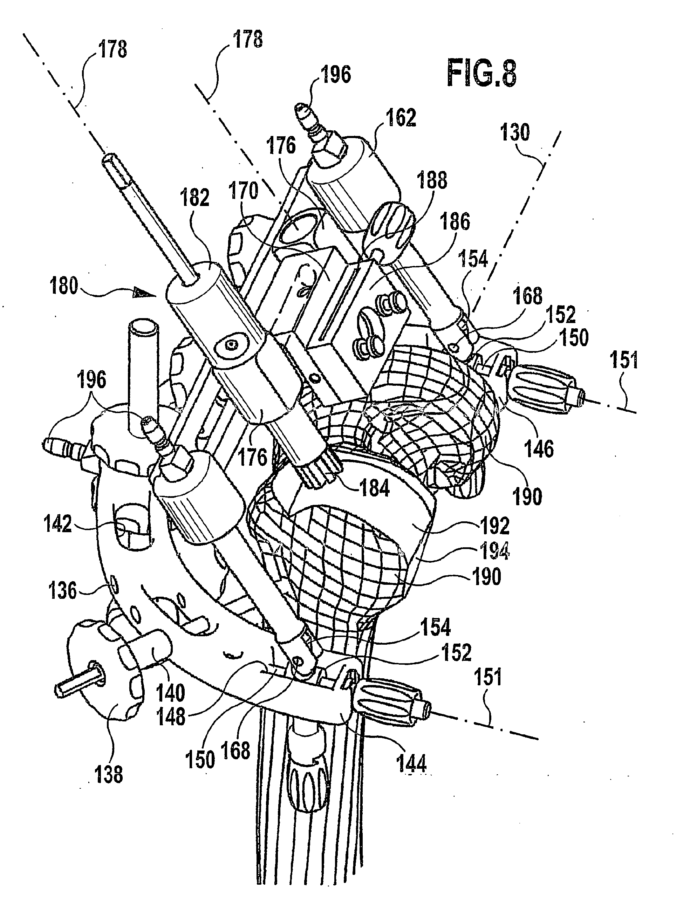

Seitlich am Lagerschlitten 170 sind zwei, jeweils in Richtung auf einen der beiden Zylinder 162 hin weisend Führungshülsen 176 angeordnet. Längsachsen 178 der Führungshülsen 176 verlaufen rechtwinklig zur Schwenkachse 130. In jede der beiden Führungshülsen 176 kann ein Bearbeitungswerkzeug, beispielsweise in Form des in den Figuren 7 und 8 dargestellten Fräsers 180, eingesetzt werden. Um Reibungen des Fräsers 180 an der Führungshülse 176 zu vermeiden, ist dieser wiederum an einem Drehlager 182 vorzugsweise kugelgelagert angeordnet, wobei sich das Drehlager 182 an einer Stirnseite der Führungshülse 176 abstützt. Der Fräser 180 weist an seinem Ende einen zylindrischen Arbeitsbereich 184 auf, so dass er als Stirnfräser und als Seitenfräser eingesetzt werden kann.

Von der Platte 156 weg weisend ist am Lagerschlitten 170 eine Schablone 186 angeordnet, die einen Führungsschlitz 188 umfasst, der die Schablone parallel zu den Längsachsen 178 durchsetzt. Er dient beispielsweise als Sägeschablone zum Führen eines nicht dargestellten Sägeblatts.

Mit dem Ausrichtinstrument 120 lassen sich zur Schwenkachse 130 konzentrische Flächen am Femur 124 oder an einem beliebigen anderen Knochen des menschlichen Körpers präparieren. Hierzu wird, wie in den Figuren 7 und 8 dargestellt, die Plattform 122 an den Knochenpins 126 und 128 am Femur 124 fixiert. Die Knochenpins 126 und 128 werden an Stellen im Femur 124 verankert, an welchen weder Sehnen noch Muskeln angewachsen sind. An einer der beiden Kondylen 190 kann mit dem Ausrichtinstrument 120 eine zylindrische Verankerungsfläche 192 präpariert werden, indem der Fräser 180 in eine der beiden Führungshülsen 176 eingeführt und dort axial unverschieblich fixiert wird. Verschwenkt man den Schwenkbügel 132 um die Schwenkachse 130, so wird bei rotierendem Arbeitsbereich 184 des als Stirnfräser wirkenden Fräsers 180 die Kondyle 190 teilweise reseziert. Es verbleibt dann die zylindrische Verankerungsfläche 192, an welcher eine nicht dargestellte künstliche Kondyle verankert werden kann. Falls erforderlich, kann an der Kondyle 190 noch ein ebener Schnitt mit Hilfe der Schablone 186 durchgeführt werden, wodurch eine ebene Schnittfläche 194 an der Kondyle 190 gebildet wird. Die Schnittfläche 194 verläuft parallel zur Schwenkachse 130.

Für einen navigationsgestützten Einsatz der Ausrichtinstrumente 10 und 120 können diese mit Kupplungszapfen 196 zum Festlegen von detektierbaren Markerelementen versehen sein. Am Ausrichtinstrument sind drei Kupplungszapfen 196 an den Zylindern 162 und am Rahmen 134 abstehend angeordnet.

## Patentansprüche

1. Chirurgische Positionier- und Haltevorrichtung (120) zum Positionieren und Halten einer Führung (176 188) für ein chirurgisches Bearbeitungswerkzeug (180), mit der Führung, mit mindestens einem Befestigungselement (126, 128) zum Fixieren in einem zu bearbeitenden Knochen (102; 124), mit mindestens einem Bearbeitungswerkzeug (180) und mit einer an dem mindestens einen Befestigungselement (126, 128) gehaltenen Plattform (122) zum Halten der Führung (176, 188), wobei die Führung (176, 188) um eine erste Drehachse (130) drehbar gelagert und derart ausgebildet ist, dass mit dem in der Führung (176, 188) geführten oder an dieser gehaltenen Bearbeitungswerkzeug (180) mindestens eine zur ersten Drehachse (130) konzentrische Fläche (192) präparierbar ist, **dadurch gekennzeichnet, dass** das Bearbeitungswerkzeug ein Stirnfräser ist und dass an der Vorrichtung (120) ein Referenzelement (196) zur Navigationskontrolle vorgesehen ist.

2. Vorrichtung nach Anspruch 1, **dadurch gekennzeichnet, dass** ein zweites Befestigungselement (126, 128) zum Fixieren in dem zu bearbeitenden Knochen (102; 124) vorgesehen ist und dass das zweite Befestigungselement (126, 128) an der Plattform (122) geführt und/oder gehalten ist.

3. Vorrichtung nach einem der voranstehenden Ansprüche, **dadurch gekennzeichnet, dass** die Plattform (122) mindestens eine Befestigungselementaufnahme (136) zum Aufnehmen des mindestens einen Befestigungselements (126, 128) aufweist.

4. Vorrichtung nach Anspruch 3, **dadurch gekennzeichnet, dass** die mindestens eine Befestigungselementaufnahme eine Bohrung (136) umfasst.

5. Vorrichtung nach einem der Ansprüche 3 oder 4, **dadurch gekennzeichnet, dass** Längsachsen von mindestens zwei Befestigungselementaufnahmen (136) parallel zueinander ausgerichtet sind.

6. Vorrichtung nach Anspruch 5, **dadurch gekennzeichnet, dass** die Längsachsen der mindestens zwei Befestigungselementaufnahmen (136) parallel oder nahezu parallel zu der ersten Drehachse (130) verlaufen.

7. Vorrichtung nach einem der voranstehenden Ansprüche, **dadurch gekennzeichnet, dass** an der Plattform (122) eine Lagerwelle (168) vorgesehen ist und dass die Lagerwelle (168) die erste Drehachse (66; 130) definiert.

8. Vorrichtung nach Anspruch 7, **dadurch gekennzeichnet, dass** die Lagerwelle (168) relativ zu dem mindestens einen Befestigungselement (126, 128) in einer ersten Verschieberichtung (151) an der Plattform (122) verschiebbar gelagert ist.

9. Vorrichtung nach einem der Ansprüche 7 oder 8, **dadurch gekennzeichnet, dass** die die Lagerwelle (168) relativ zu dem mindestens einen Befestigungselement (126, 128) in einer zweiten Verschieberichtung an der Plattform (122) verschiebbar gelagert ist.

10. Vorrichtung nach einem der Ansprüche 7 bis 9, **dadurch gekennzeichnet, dass** die Lagerwelle relativ zu dem mindestens einen Befestigungselement um eine zweite Drehachse an der Plattform schwenkbar gelagert ist.

11. Vorrichtung nach Anspruch 10, **dadurch gekennzeichnet, dass** die erste und die zweite Drehachse rechtwinklig zueinander orientiert sind.

12. Vorrichtung nach einem der Ansprüche 7 bis 11, **dadurch gekennzeichnet, dass** die Vorrichtung (120) einen um die erste Drehachse (130) drehbar gelagerten Gelenkarm (132) umfasst, dass ein Ende des Gelenkarms (132) an der Lagerwelle (168) um die erste Drehachse (130) drehbar gelagert ist und dass ein anderes Ende (170) des Gelenkarms (132) die Führung (176, 188) trägt.

13. Vorrichtung nach einem der voranstehenden Ansprüche, **dadurch gekennzeichnet, dass** die Führung (176) eine Hülse (182) zum Aufnehmen des Bearbeitungswerkzeugs (180) umfasst.

14. Vorrichtung nach Anspruch 13, **dadurch gekennzeichnet, dass** die Hülse (182) am Gelenkarm (132) rotierbar gelagert ist.

15. Vorrichtung nach einem der Ansprüche 7 bis 14 soweit diese direkt oder indirekt auf Anspruch 7 rückbezogen sind, **dadurch gekennzeichnet, dass** an der Plattform ein erster Linearantrieb vorgesehen ist zum Verschieben der Lagerwelle relativ zu dem mindestens einen Befestigungselement in der ersten Verschieberichtung .

16. Vorrichtung nach Anspruch 15, **dadurch gekennzeichnet, dass** der erste Linearantrieb ein Spindeltrieb mit einer ersten Gewindespindel und einem ersten Antriebsknopf ist und dass eine Längsachse der ersten Gewindespindel die erste Verschieberichtung definiert.

17. Vorrichtung nach einem der Ansprüche 9 bis 16, **dadurch gekennzeichnet, dass** an der Plattform ein zweiter Linearantrieb vorgesehen ist zum Verschieben der Lagerwelle relativ zu dem mindestens einen Befestigungselement in der zweiten Verschieberichtung.

18. Vorrichtung nach Anspruch 17, **dadurch gekennzeichnet, dass** der zweite Linearantrieb ein zweiter Spindeltrieb mit einer zweiten Gewindespindel und einem zweiten Antriebsknopf ist und dass eine Längsachse der zweiten Gewindespindel die zweite Verschieberichtung definiert.

19. Vorrichtung nach Anspruch 18, **dadurch gekennzeichnet, dass** die Längsachse der zweiten Gewindespindel die zweite Drehachse definiert.

20. Vorrichtung nach einem der Ansprüche 10 bis 19 soweit diese direkt oder indirekt auf Anspruch 7 rückbezogen sind, **dadurch gekennzeichnet, dass** an der Plattform ein Exzenterantrieb vorgesehen ist zum Verschwenken der Lagerwelle relativ zu dem mindestens einen Befestigungselement um die zweite Drehachse.

21. Vorrichtung nach Anspruch 20, **dadurch gekennzeichnet, dass** der Exzenterantrieb einen exzentrisch um eine dritte Drehachse gelagerten Drehkörper umfasst und dass die dritte Drehachse parallel zur zweiten Drehachse verläuft.

22. Vorrichtung nach einem der voranstehenden Ansprüche, **dadurch gekennzeichnet, dass** das mindestens eine Befestigungselement (126, 128) von der ersten Drehachse (130) beabstandet ist.

23. Vorrichtung nach einem der voranstehenden Ansprüche, **dadurch gekennzeichnet, dass** die Führung (176, 188) relativ zur Plattform (122) in einer Drehstellung feststellbar ist.

24. Vorrichtung nach einem der voranstehenden Ansprüche, **dadurch gekennzeichnet, dass** die Führung (176) eine vierte Drehachse (178) definiert.

25. Vorrichtung nach einem der Ansprüche 9 bis 24, **dadurch gekennzeichnet, dass** eine Breite der Plattform in der zweiten Verschieberichtung maximal 30 mm beträgt.

26. Vorrichtung nach einem der voranstehenden Ansprüche, **dadurch gekennzeichnet, dass** ein Abstand der Führung von der ersten Drehachse in einem Bereich von 15 mm bis 50 mm liegt.

27. Vorrichtung nach einem der Ansprüche 12 bis 26, **dadurch gekennzeichnet, dass** ein Satz unterschiedlich langer Gelenkarme vorgesehen ist und dass jeder Gelenkarm einen anderen Abstand zwischen der ersten Drehachse und der Führung aufweist.

28. Vorrichtung nach einem der voranstehenden Ansprüche, **dadurch gekennzeichnet, dass** die Führung (176, 188) in einer Richtung parallel zur ersten Drehachse (130) relativ zur Plattform (122) verschiebbar ist.

29. Vorrichtung nach einem der Ansprüche 24 bis 28, **dadurch gekennzeichnet, dass** eine von der Führung (176) definierte vierte Drehachse (178) rechtwinklig zur ersten Drehachse (130) verläuft.

## Claims

1. Surgical positioning and holding device (120) for positioning and holding a guide (176, 188) for a surgical machining tool (180), with the guide, with at least one securing element (126, 128) for fixation to a bone (102; 124) to be machined, with at least one machining tool (180), and with a platform (122) held on the at least one securing element (126, 128) for holding the guide (176, 188), the guide (176, 188) being mounted for rotation about a first axis of rotation (130) and being designed such that at least one surface (192) concentric with the first axis of rotation (130) may be prepared with the machining tool (180) guided in or held on the guide (176, 188), **characterized in that** the machining tool is a face milling cutter, and **in that** a reference element (196) for navigation control is provided on the device (120).

2. Device in accordance with claim 1, **characterized in that** a second securing element (126, 128) is provided for fixation to the bone (102; 124) to be machined, and **in that** the second securing element (126, 128) is guided and/or held on the platform (122).

3. Device in accordance with any one of the preceding claims, **characterized in that** the platform (122) comprises at least one securing element receptacle (136) for receiving the at least one securing element (126, 128).

4. Device in accordance with claim 3, **characterized in that** the at least one securing element receptacle comprises a bore (136).

5. Device in accordance with claim 3 or 4, **characterized in that** longitudinal axes of at least two securing element receptacles (136) are aligned parallel to each other.

6. Device in accordance with claim 5, **characterized in that** the longitudinal axes of the at least two securing element receptacles (136) extend parallel or almost parallel to the first axis of rotation (130).

7. Device in accordance with any one of the preceding claims, **characterized in that** a bearing shaft (168) is provided on the platform (122), and **in that** the bearing shaft (168) defines the first axis of rotation (66; 130).

8. Device in accordance with claim 7, **characterized in that** the bearing shaft (168) is mounted on the platform (122) for displacement in a first direction of displacement (151) relative to the at least one securing element (126, 128).

9. Device in accordance with claim 7 or 8, **characterized in that** the bearing shaft (168) is mounted on the platform (122) for displacement in a second direction of displacement relative to the at least one securing element (126, 128).

10. Device in accordance with any one of claims 7 to 9, **characterized in that** the bearing shaft is mounted on the platform for pivotal movement about a second axis of rotation relative to the at least one securing element.

11. Device in accordance with claim 10, **characterized in that** the first and second axes of rotation are oriented at right angles to each other.

12. Device in accordance with any one of claims 7 to 11, **characterized in that** the device (120) comprises an articulated arm (132) mounted for rotation about the first axis of rotation (130), **in that** one end of the articulated arm (132) is mounted on the bearing shaft (168) for rotation about the first axis of rotation (130), and **in that** another end (170) of the articulated arm (132) carries the guide (176, 188).

13. Device in accordance with any one of the preceding claims, **characterized in that** the guide (176) comprises a sleeve (182) for receiving the machining tool (180).

14. Device in accordance with claim 13, **characterized in that** the sleeve (182) is mounted for rotation on the articulated arm (132).

15. Device in accordance with any one of claims 7 to 14, insofar as these refer back directly or indirectly to claim 7, **characterized in that** a first linear drive is provided on the platform for displacing the bearing shaft in the first direction of displacement relative to the at least one securing element.

16. Device in accordance with claim 15, **characterized in that** the first linear drive is a spindle drive with a first threaded spindle and a first drive knob, and **in that** a longitudinal axis of the first threaded spindle defines the first direction of displacement.

17. Device in accordance with any one of claims 9 to 16, **characterized in that** a second linear drive is provided on the platform for displacing the bearing shaft in the second direction of displacement relative to the at least one securing element.

18. Device in accordance with claim 17, **characterized in that** the second linear drive is a second spindle drive with a second threaded spindle and a second drive knob, and **in that** a longitudinal axis of the second threaded spindle defines the second direction of displacement.

19. Device in accordance with claim 18, **characterized in that** the longitudinal axis of the second threaded spindle defines the second axis of rotation.

20. Device in accordance with any one of claims 10 to 19, insofar as these refer back directly or indirectly to claim 7, **characterized in that** an eccentric drive is provided on the platform for pivoting the bearing shaft about the second axis of rotation relative to the at least one securing element.

21. Device in accordance with claim 20, **characterized in that** the eccentric drive comprises a rotational member mounted eccentrically about a third axis of rotation, and **in that** the third axis of rotation extends parallel to the second axis of rotation.

22. Device in accordance with any one of the preceding claims, **characterized in that** the at least one securing element (126, 128) is spaced from the first axis of rotation (130).

23. Device in accordance with any one of the preceding claims, **characterized in that** the guide (176, 188) is securable in a rotational position relative to the platform (122).

24. Device in accordance with any one of the preceding claims, **characterized in that** the guide (176) defines a fourth axis of rotation (178).

25. Device in accordance with any one of claims 9 to 24, **characterized in that** a width of the platform in the second direction of displacement is 30 mm at maximum.

26. Device in accordance with any one of the preceding claims, **characterized in that** a spacing of the guide from the first axis of rotation lies in a range of from 15 mm to 50 mm.

27. Device in accordance with any one of claims 12 to 26, **characterized in that** a set of articulated arms of different lengths is provided, and **in that** each articulated arm has a different spacing between the first axis of rotation and the guide.

28. Device in accordance with any one of the preceding claims, **characterized in that** the guide (176, 188) is displaceable in a direction parallel to the first axis of rotation (130) relative to the platform (122).

29. Device in accordance with any one of claims 24 to 28, **characterized in that** a fourth axis of rotation (178) defined by the guide (176) extends at right angles to the first axis of rotation (130).

## Revendications

1. Dispositif chirurgical de positionnement et de maintien (120) pour positionner et maintenir un guide (176, 188) destiné à un outil d'usinage chirurgical (180), le dispositif comprenant le guide, au moins un élément de fixation (126, 128) pour la fixation dans un os à usiner (102; 124), comprenant également au moins un outil d'usinage (180) et une plateforme (122) qui est maintenue sur ledit au moins un élément de fixation (126, 128) pour maintenir le guide (176, 188), le guide (176, 188) étant monté rotatif autour d'un premier axe de rotation (130) et étant configuré de manière à pouvoir préparer, avec l'outil d'usinage (180) guidé dans le guide (176, 188) ou maintenu sur celui-ci, au moins une surface (192) concentrique au premier axe de rotation (130),
**caractérisé en ce que** l'outil d'usinage est une fraise en bout à surfacer, et **en ce que** sur le dispositif (120) est prévu un élément de référence (196) pour le contrôle de navigation.

2. Dispositif selon la revendication 1, **caractérisé en ce qu'**il est prévu un deuxième élément de fixation (126, 128) pour la fixation dans l'os à usiner (102; 124), et **en ce que** le deuxième élément de fixation (126, 128) est guidé et/ou maintenu sur la plateforme (122).

3. Dispositif selon l'une des revendications précédentes, **caractérisé en ce que** la plateforme (122) présente au moins un logement de réception d'élément de fixation (136) destiné à recevoir ledit au moins un élément de fixation (126, 128).

4. Dispositif selon la revendication 3, **caractérisé en ce que** ledit au moins un logement de réception d'élément de fixation comprend un alésage (136).

5. Dispositif selon l'une des revendications 3 ou 4, **caractérisé en ce que** des axes longitudinaux d'au moins deux logements de réception d'élément de fixation (136) sont orientés parallèlement les uns aux autres.

6. Dispositif selon la revendication 5, **caractérisé en ce que** les axes longitudinaux desdits au moins deux logements de réception d'élément de fixation (136) s'étendent parallèlement ou pratiquement parallèlement au premier axe de rotation (130).

7. Dispositif selon l'une des revendications précédentes, **caractérisé en ce que** sur la plateforme (122) est prévu un arbre de palier (168), et **en ce que** l'arbre de palier (168) définit le premier axe de rotation (66; 130).

8. Dispositif selon la revendication 7, **caractérisé en ce que** l'arbre de palier (168) est monté sur la plateforme (122) de manière à pouvoir coulisser par rapport audit au moins un élément de fixation (126, 128), dans une première direction de coulissement (151).

9. Dispositif selon l'une des revendications 7 ou 8, **caractérisé en ce que** l'arbre de palier (168) est monté sur la plateforme (122) de manière à pouvoir coulisser par rapport audit au moins un élément de fixation (126, 128), dans une deuxième direction de coulissement.

10. Dispositif selon l'une des revendications 7 à 9, **caractérisé en ce que** l'arbre de palier est monté sur la plateforme de manière à pouvoir pivoter autour d'un deuxième axe de rotation, par rapport audit au moins un élément de fixation.

11. Dispositif selon la revendication 10, **caractérisé en ce que** le premier et le deuxième axe de rotation sont orientés perpendiculairement l'un par rapport à l'autre.

12. Dispositif selon l'une des revendications 7 à 11, **caractérisé en ce que** le dispositif (120) comprend un bras d'articulation (132) monté rotatif autour du premier axe de rotation (130), **en ce qu'**une extrémité du bras d'articulation (132) est montée sur l'arbre de palier (168), de manière rotative autour de l'axe de rotation (130), et **en ce qu'**une autre extrémité (170) du bras d'articulation (132) porte le guide (176, 188).

13. Dispositif selon l'une des revendications précédentes, **caractérisé en ce que** le guide (176) comprend un fourreau (182) destiné à recevoir l'outil d'usinage (180).

14. Dispositif selon la revendication 13, **caractérisé en ce que** le fourreau (182) est monté rotatif sur le bras d'articulation (132).

15. Dispositif selon l'une des revendications 7 à 14 dans la mesure où celles-ci sont rattachées directement ou indirectement à la revendication 7, **caractérisé en ce que** sur la plateforme est prévu un premier entraînement linéaire pour faire coulisser l'arbre de palier par rapport audit au moins un élément de fixation, dans la première direction de coulissement.

16. Dispositif selon la revendication 15, **caractérisé en ce que** le premier entraînement linéaire est un entraînement à vis avec une première vis filetée et un premier bouton d'entraînement, et **en ce qu'**un axe longitudinal de la première vis filetée définit la première direction de coulissement.

17. Dispositif selon l'une des revendications 9 à 16, **caractérisé en ce que** sur la plateforme est prévu un deuxième entraînement linéaire pour faire coulisser l'arbre de palier par rapport audit au moins un élément de fixation, dans la deuxième direction de coulissement.

18. Dispositif selon la revendication 17, **caractérisé en ce que** le deuxième entraînement linéaire est un deuxième entraînement à vis avec une deuxième vis filetée et un deuxième bouton d'entraînement, et **en ce qu'**un axe longitudinal de la deuxième vis filetée définit la deuxième direction de coulissement.

19. Dispositif selon la revendication 18, **caractérisé en ce que** l'axe longitudinal de la deuxième vis filetée définit le deuxième axe de rotation.

20. Dispositif selon l'une des revendications 10 à 19 dans la mesure où celles-ci sont rattachées directement ou indirectement à la revendication 7, **caractérisé en ce que** sur la plateforme est prévu un entraînement excentrique pour faire pivoter l'arbre de palier par rapport audit au moins un élément de fixation, autour du deuxième axe de rotation.

21. Dispositif selon la revendication 20, **caractérisé en ce que** l'entraînement excentrique comprend un corps rotatif monté de manière excentrée autour d'un troisième axe de rotation, et **en ce que** le troisième axe de rotation s'étend parallèlement au deuxième axe de rotation.

22. Dispositif selon l'une des revendications précédentes, **caractérisé en ce que** ledit au moins un élément de fixation (126, 128) est espacé du premier axe de rotation (130).

23. Dispositif selon l'une des revendications précédentes, **caractérisé en ce que** le guide (176, 188) peut être immobilisé par rapport à la plateforme (122), dans une position de rotation.

24. Dispositif selon l'une des revendications précédentes, **caractérisé en ce que** le guide (176) définit un quatrième axe de rotation (178).

25. Dispositif selon l'une des revendications 9 à 24, **caractérisé en ce qu'**une largeur de la plateforme dans la deuxième direction de coulissement vaut au maximum 30 mm.

26. Dispositif selon l'une des revendications précédentes, **caractérisé en ce qu'**une distance d'espacement du guide par rapport au premier axe de rotation, se situe dans une plage de 15 mm à 50 mm.

27. Dispositif selon l'une des revendications 12 à 26, **caractérisé en ce qu'**il est prévu un jeu de bras d'articulation de longueur différente, et **en ce que** chaque bras d'articulation présente une autre distance d'espacement entre le premier axe de rotation et le guide.

28. Dispositif selon l'une des revendications précédentes, **caractérisé en ce que** le guide (176, 188) peut coulisser par rapport à la plateforme (122), dans une direction parallèle au premier axe de rotation (130).

29. Dispositif selon l'une des revendications 24 à 28, **caractérisé en ce qu'**un quatrième axe de rotation (178) défini par le guide (176) s'étend perpendiculairement au premier axe de rotation (130).
